# EUROPÄISCHE PATENTSCHRIFT

(11) **EP 0 486 979 B1**
(45) Veröffentlichungstag und Bekanntmachung des Hinweises auf die Patenterteilung: **11.10.1995**
(21) Anmeldenummer: 91119590.7
(22) Anmeldetag: 16.11.1991
(51) Int. Cl.: A61B 5/042, H01R 11/26

(54) **Führungssonde**
Guiding probe
Sonde de guidage

(30) Priorität: 21.11.1990 DE 9015857 U
(43) Veröffentlichungstag der Anmeldung: 27.05.1992
(73) Patentinhaber: B. Braun Melsungen AG, 34209 Melsungen (DE)
(72) Erfinder: Maier, Hans-Otto, Dr., W-3503 Lohfelden (DE)
(74) Vertreter: Selting, Günther, Dipl.-Ing.

(56) Entgegenhaltungen:
- EP-A- 0 367 472
- DE-A- 3 417 479
- FR-E- 95 424
- US-A- 4 354 723
- US-A- 4 552 127
- "Plazierung und Lagekorrektur von zentralen Venenkathetern mit der Seldinger-Spirale"; Anaesthesist 29, 498 - 503 (1980).

## Beschreibung

Die Erfindung bezieht sich auf eine Führungssonde zur Verlegung eines flexiblen Katheters nach der Seldinger-Technik.

Derartige Führungssonden haben die Aufgabe, die Verlegung von Kathetern zu erleichtern, indem sie nach der mit Hilfe einer Punktionskanüle durchgeführten Punktion durch diese Punktionskanüle in das Gefäßlumen eingeführt werden, und anschließend der Katheter über die als "Pfadfinder" dienende Führungssonde in die angestrebte Position vorgeschoben wird. Diese Methode der Plazierung von Kathetern wird als "Seldinger-Technik" bezeichnet. Die Führungssonde wird bisher unter Durchleuchtungskontrolle eingebracht.

Zur Überwindung von Hindernissen auf den Vorschubweg der Führungssonde ist ihre Spitze sehr flexibel und vorzugsweise ist sie mit einer halbkreisförmigen Krümmung versehen. Die verbleibende Länge des Drahtes, die bis zu 100 cm lang sein kann, ist zur Erzielung unterschiedlicher Flexibilität für verschiedene Einsatzzwecke in vielfältiger Weise modifiziert worden. Beispielsweise ist er aus einer Kombination einer Litze aus verseilt umeinandergewickelten Drähten und einem Rohrschaft aufgebaut (EP-A-200 919). Auch ist es bekannt, abwechselnd flexible, verseilte Abschnitte mit steifen Vollmaterialabschnitten zu einem Draht zusammenzusetzen (DE-GM 86 08 051) oder eine einteilige Drahtseele durch partielle Querschnittsverjüngungen in Abschnitte unterschiedlicher Steifigkeit zu unterteilen (DE-GM 89 00 077).

Unabhängig von dem Aufbau des Drahtes ist immer sein patientenfernes Ende gerade und vorsprungslos sowie von jeglichen Ansatzstücken frei, damit nach Verlegung der Führungssonde über ihr patientenfernes Ende der Katheter auf die Führungssonde aufgefädelt werden kann. Dieses Erfordernis der Freihaltung des patientenfernen Endes des Drahtes machte es bisher notwendig, die Positionierung unter Röntgen-Durchleuchtungskontrolle durchzuführen und auch zur Lagekorrektur das Röntgenbild zur Hilfe zu nehmen. Hierin besteht ein wesentlicher Unterschied zu Katheterbestecken, bei denen herstellerseitig ein Führungsstab als lumenfüllender Mandrin in den Katheter eingesetzt ist, um mit diesem gemeinsam in das Blutgefäß eingeführt zu werden. Der Katheter ist mit dem Mandrin durch Kupplungsstücke an ihren patientenfernen Enden verbunden, die den problemlosen Anschluß einer Ableitung zu einem EKG-Gerät ermöglichen, um aus EKG-Kurven Rückschlüsse über die Position der Spitze des Katheters zu ziehen und damit die schädlichen Strahlenbelastungen der Röntgenkontrolle zu vermeiden (DE-GM 85 09 649).

Eine weitere Kathetervorrichtung mit Mandrin beschreibt US-A-4 552 127. Das patientennahe Ende des elektrisch leitfähigen Mandrins ist mit einer Elektrode in der Spitze eines Ballons verbunden und an dem patientenfernen Ende ist ein Kopfstück unlösbar befestigt, das einen axialen Gewindestift trägt, auf den ein Handrädchen soweit aufgeschraubt ist, daß ein Ende des Gewindestiftes nach außen übersteht. Das Handrädchen ist auf dem Gewindestift undrehbar fixiert und an das Ende ist eine Federklemme einer EKG-Leitung lösbar anklemmbar. Mit Hilfe des Handrädchens wird der Mandrin gedreht, um den Ballon zu verdrillen und auf den Mandrin aufzuwickeln, damit sein Durchmesser zur Erleichterung der Einführung in die Aorta verringert wird. Nach der Einführung der aus Mandrin und Ballonkatheter bestehenden Einheit wird das Handrädchen entgegengesetzt gedreht, damit der Ballon von dem Mandrin abgewickelt wird und aufgepumpt werden kann. Nach der Verlegung des Ballonkatheters wird die EKG-Leitung angeklemmt, um die Funktion des intra-aortalen Ballons mit dem Herzen mittels EKG-Signalen zu synchronisieren.

Dieser bekannte intra-aortale Ballonkatheter dient einem besonderen Zweck, der nichts mit der Seldinger-Technik zur Erleichterung der Verlegung sehr langer Katheter zu tun hat. Der fest zwischen der Katheterspitze und dem Kopfstück eingespannte Mandrin ist nicht als Seldinger-Führungssonde benutzbar, die zunächst als "Pfadfinder" in ein Gefäß vorgeschoben wird und über die nach der Art einer Schiene ein langer Katheter nachgeschoben werden kann. Auch ist die Federklemme nicht geeignet zum Ansetzen an eine Seldinger-Führungssonde, weil ihre zangenartigen Klemmorgane und ihre sperrige Gestalt dem Anwender eine feinfühlige Führung der Führungssonde unmöglich machen würden.

Der Erfindung liegt die Aufgabe zugrunde, eine Plazierung und Lagekorrektur von Führungssonden für die Katheterverlegung nach der Seldinger-Technik unter EKG-Kontrolle zu ermöglichen.

Diese Aufgabe wird erfindungsgemäß durch die Merkmale des Anspruchs 1 gelost.

Wenn nach der Gefäßpunktion durch die Punktionskanüle die Führungssonde in die Hohlvene hineingeschoben und die Punktionskanüle über das patientenferne Ende von der Führungssonde abgezogen worden ist, wird das Kontaktelement an das patientenferne Ende der Führungssonde angesetzt, und es kann über die an das Anschlußteil angeschlossene EKG-Leitung ein intrakardiales EKG abgeleitet werden, das Rückschlüsse auf die Lage der Spitze der Führungssonde zuläßt. Nach korrekter Plazierung der Spitze wird das Kontaktelement von dem patientenfernen Ende der Führungssonde abgezogen, und es wird der Katheter über den "Pfadfinder" bis zum Zielort vorgeschoben. Da die Spitze des Katheters der korrekt plazierten Führungssonde folgt, sind Fehllagen des Katheters nahezu ausgeschlossen. Bei einer anderen Vorgehensweise wird das elektrische Kontaktelement erst dann auf den Draht der Führungssonde aufgesteckt, wenn nach Abziehen der Punktionskanüle der Katheter bereits auf die Führungssonde aufgeschoben wurde, und es setzt die EKG-Ableitung erst ein, wenn die Katheterspitze in die Nähe der Spitze der Führungssonde gelangt, die zum Zielort vordringt. Sobald die EKG-Kurve die korrekte Verlegung der Katheterspitze anzeigt, wird die Führungssonde aus den Katheter herausgezogen, und es kann an den Katheteransatz in üblicher Weise ein Infusionsschlauch oder dgl. angeschlossen werden. Die insbesondere bei zu überwindenden Hindernissen im Gefäßlumen bevorzugte Katheterverlegung nach der Seldinger-Technik wird für den Patienten sicherer, weil Strahlenbelastungen durch Röntgenaufnahmen entfallen. Auch erspart der Wegfall von Röntgenaufnahmen Kosten. Ferner wird die Handhabung von Führungssonde und Katheter für den Anwender erleichtert, da er nicht mehr ein Röntgenbild scharf beobachten, sondern nur auf EKG-Kurvensignale reagieren muß.

In vorteilhafter Ausgestaltung der Erfindung ist vorgesehen, daß der Hohlraum mit der Einstecköffnung für das Drahtende konisch ist, daß die Einstecköffnung dem Anschlußteil gegenüberliegt und daß der Hohlraum sich in Richtung des Anschlußteiles verjüngt und auf seiner Innenfläche tannenbaumartig profiliert ist. Auf diese Weise wird eine gedrungene Bauform der Klemmbuchse und ihre Anpassung an Drähte verschiedener Durchmesser erreicht. Jede Etage der tannenbaumartig profilierten Innenfläche bewirkt für den zugeordneten Drahtdurchmesser eine klemmende Halterung in der Klemmbuchse.

Vorteilhaftenweise ist die Klemmbuchse mit den Anschlußteil in Stiftform einstückig aus Metall gefertigt und in einem rohrförmigen Kunststoff- Gehäuse des Kontaktelementes angeordnet. Das Gehäuse weist an einen Ende einen Hülsenansatz auf, dessen Umfangswandung das Anschlußteil mit Abstand umgibt. Sowohl die Klemmbuchse als auch das Anschlußteil sind auf diese Weise nach außen abgeschirmt, und der Hülsenansatz mit den zentral in diesen vorgesehenen Anschlußteil bildet einen Stecker, in den eine elektrische Kupplung einer EKG-Leitung, die zu einen EKG-Gerät führt, eingeschoben werden kann.

Das Gehäuse weist an den der Einstecköffnung des Hohlraumes benachbarten Ende einen Rohrstutzen mit einem geraden Kanal zur Führung des patientenfernen Endes des Drahtes auf. Der Rohrstutzen erleichtert das gezielte Einführen des Drahtendes in die Einstecköffnung der Klemmbuchse. Auch dient er der Stabilisierung der Steckverbindung zur Gewährleistung zuverlässiger EKG-Signale. Ferner erhält das Gehäuse durch den Rohrstutzen eine handliche Länge, die das Anfassen beim Zusammenstecken der Teile, vereinfacht, was insbesondere deshalb wichtig ist, weil die in das Gefäß hineinragende Führungssonde möglichst unbeweglich liegenbleiben soll.

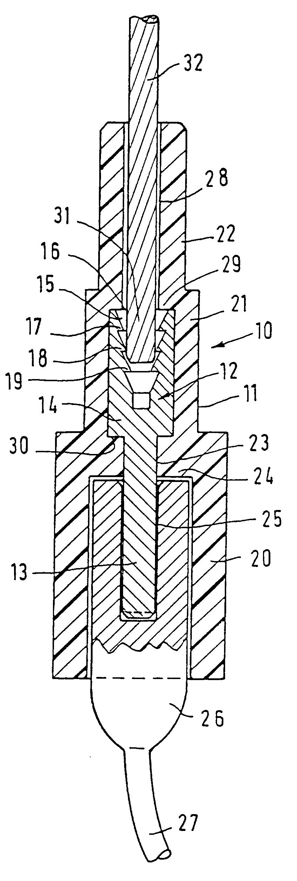

In der Zeichnung ist ein Ausführungsbeispiel der Erfindung schematisch dargestellt.

Die einzige Figur zeigt einen Längsschnitt durch ein elektrisches Kontaktelement im Anwendungszustand.

Ein elektrisches Kontaktelement 10 besteht im wesentlichen aus einen rohrförmigen Gehäuse 11 aus elektrisch nichtleitendem Kunststoffmaterial, das eine Klemmbuchse 12 aus Metall enthält. Die Klemmbuchse 12 hat einen kreiszylindrischen länglichen Körper 14, der über den größten Teil seiner Länge einen koaxialen konischen Hohlraum 15 enthält, der durch eine Einstecköffnung 16 in der einen Stirnfläche des Körpers 14 zugänglich und am anderen Ende geschlossen ist. Von der der Einstecköffnung 16 gegenüberliegenden Stirnfläche des Körpers 14 geht senkrecht ein Anschlußteil 13 für eine EKG-Leitung 27 aus, das als kreiszylindrischer Kontaktstift gebildet ist, dessen Achse auf der Mittelachse des Hohlraumes 15 liegt. Die schräge geschlossene Innenfläche des konischen Hohlraumes 15 ist tannenbaumartig profiliert. Dadurch entstehen axial aufeinanderfolgende scharfkantige Ringe 17, 18, 19, die Kreisdurchlässe verschiedener Durchmesser begrenzen. Da der Hohlraum 15 sich von der Einstecköffnung 16 in Richtung des Anschlußteiles 13 verjüngt, ist der Durchlaß des Ringes 17 am größten und der Durchlaß des Ringes 19 am kleinsten.

Das rohrförmige einteilige Gehäuse 11 besteht aus drei koaxial aufeinanderfolgenden Abschnitten 20, 21, 22, die jeweils kreiszylindrisch gestaltet sind. Der zwischen den beiden Abschnitten 20 und 22 befindliche Abschnitt 21 bildet eine kreiszylindrische Kammer, in der der Körper 14 der Klemmbuchse 12 passend untergebracht ist, so daß die Klemmbuchse 12 in den Gehäuse 11 festsitzt. Das mit dem Körper 14 einstückig ausgebildete Anschlußteil 13 ragt durch eine Bohrung 23 in einer Querwand 24 des Gehäuses 11 hindurch und endet frei im kreiszylindrischen Innenraum 25 eines Hülsenansatzes, der den Abschnitt 20 darstellt. Die Innenfläche des Hülsenansatzes ist glatt oder mit einem Gewindegang versehen. Das Ende des Anschlußteiles 13 ist zu der Öffnung des Hülsenansatzes nach innen zurückgesetzt. Anschlußteil 13 und Hülsenansatz bilden eine Steckerbuchse, in die ein Kupplungsstück 26 der EKG-Leitung 27 einsteckbar ist.

Der andere Abschnitt 22 des Gehäuses 11 ist als Rohrstutzen mit einem geraden Kanal 28 gestaltet. Der Kanal 28 ist an beiden Enden offen und mündet innen in die Einstecköffnung 16 des Hohlraumes 15 der Klemmbuchse 12. An der Einmündung des Kanals 28 in den Hohlraum 15 befindet sich eine Ringschulter 29, die einer parallelen Ringschulter 30 an der Einmündung der Bohrung 23 in den Hohlraum 15 gegenüberliegt. Die beiden Ringschultern 29 und 30 liegen gegen die Stirnseiten des Körpers 14 der Klemmbuchse 12 an und halten diese fest.

Durch den Kanal 28 des Rohrstutzenabschnittes 22 wird das gerade vorsprungslose patientenferne Ende 31 eines elektrisch leitfähigen Drahtes 32 einer mit ihrem anderen Ende in eine Hohlvene eingeführten Führungssonde in den Hohlraum 15 der Klemmbuchse 12 geschoben. Je nach dem Durchmesser des Drahtes 32 bleibt das Ende 31 in dem Ring 17, 18 oder 19 stecken und wird von seinen scharfen inneren Kanten klemmend festgehalten. Dadurch wird eine elektrische Verbindung zwischen dem Draht 32 und der EKG-Leitung 27 hergestellt, und die elektrischen Herzimpulse werden durch den Draht 32 direkt von seiner Spitze zu einem an die Leitung 27 angeschlossenen EKG-Gerät geleitet. Von den EKG-Kurven auf einem Monitor läßt sich die Lage der Drahtspitze ablesen und ggf. korrigieren.

## Patentansprüche

1. Führungssonde, bestehend aus
einem elektrisch leitfähigen Draht (32) mit einem geraden, vorsprungslosen patientenfernen Ende (31), über das ein flexibler Katheter in die Hohlvene hinein vorschiebbar ist,
einem elektrischen Kontaktelement (10) mit einer einen Hohlraum (15) mit Einstecköffnung (16) aufweisenden Klemmbuchse (12) zum Aufklemmen auf das patientenferne Ende (31) des Drahtes (32)
und einem mit der Klemmbuchse (12) verbundenen Anschlußteil (13) zum Anschluß einer EKG-Leitung (27), so daß der Draht (32) unter EKG-Kontrolle verlegbar und das Kontaktelement (10) von seinem patientenfernen Ende (31) abnehmbar ist, damit der Katheter über das Ende (31) des Drahtes (32) in Richtung des Patienten vorschiebbar ist.

2. Führungssonde nach Anspruch 1, dadurch gekennzeichnet, daß der Hohlraum (15) mit der Einstecköffnung (16) für das Drahtende (31) konisch ist,
daß die Einstecköffnung (16) dem Anschlußteil (13) gegenüberliegt
und daß der Hohlraum (15) sich in Richtung des Anschlußteiles verjüngt und auf seiner Innenfläche tannenbaumartig (17,18,19) profiliert ist.

3. Führungssonde nach Anspruch 1 oder 2, dadurch gekennzeichnet, daß die Klemmbuchse (12) mit dem Anschlußteil (13) in Stiftform einstückig aus Metall gefertigt und in einem rohrförmigen Kunststoff-Gehäuse (11) des Kontaktelementes (10) angeordnet ist
und daß das Gehäuse (11) an einem Ende einen Hülsenansatz (20) aufweist, dessen Umfangswandung das Anschlußteil (13) mit Abstand umgibt.

4. Führungssonde nach Anspruch 3, dadurch gekennzeichnet, daß das Gehäuse (11) an dem der Einstecköffnung (16) des Hohlraumes (15) benachbarten Ende einen Rohrstutzen (22) mit einem geraden Kanal (28) zur Führung des patientenfernen Endes (31) des Drahtes (32) aufweist.

## Claims

1. A guide probe, comprising
an electrically conductive wire (32) having a straight, projection-free distal end (31) adapted to have a flexible catheter advanced thereover into the vena cava,
an electric contact element (10) having a clamping bushing (12), including a cavity (15) with an insertion opening (16), for clampingly engaging the distal end (31) of the wire (32),
and a connection piece (13) associated with the clamping bushing (12) for connecting an ECG lead (27) in such a manner that the wire (32) is positionable under ECG control and the contact element (10) is removable from the distal end (31) of the wire to thereby permit the advancement of the catheter over the end (31) of the wire (32) in the direction of the patient.

2. The guide probe according to claim 1, characterized in that the cavity (15) having the insertion opening (16) for the wire end (31) formed therein is conical,
that the insertion opening (16) is arranged opposite to the connection piece (13),
and that the cavity (15) tapers towards the connection piece and has its inner surface shaped like a fir-tree (17,18,19).

3. The guide probe according to claim 1 or 2, characterized in that the clamping bushing (12) is integrally formed from metal with the pin-shaped connection piece (13) and is arranged in a tubular plastic housing (11) of the contact element (10),
and that one end of the housing (11) is provided with a sleeve extension (20) having its peripheral wall surrounding the connection piece (13) at a distance.

4. The guide probe according to claim 3, characterized in that the housing (11) at the end adjacent to the insertion opening (16) of the cavity (15) is provided with a tube connector (22) having a linear channel (28) for guiding the distal end (31) of the wire (32).

## Revendications

1. Sonde de guidage constituée
d'un fil (32) électriquement conducteur présentant une extrémité (31) éloignée du patient, rectiligne et sans saillies, par-dessus laquelle un cathéter flexible peut etre avancé à l'interieur de la vene,
d'un élément de contact électrique (10) comportant une douille de connexion par pincement (12) à cavité (15) présentant une ouverture d'insertion (16), destinée à être pincée sur l'extrémité (31) du fil (32), éloignée du patient,
et d'une partie de connexion (13) reliée à la douille de connexion par pincement (12), pour le raccordement d'un conducteur d'E.C.G (27), de sorte que le fil (32) peut être posé sous contrôle E.C.G., et de sorte que l'élément de contact (10) peut être retiré de son extrémité (31) éloignée du patient, pour que le cathéter puisse être avancé par-dessus l'extrémité (31) du fil (32), en direction du patient.

2. Sonde de guidage selon la revendication 1, caractérisée en ce que la cavité (15) présentant l'ouverture d'insertion (16) pour l'extrémité (31) du fil, est conique, en ce que l'ouverture d'insertion (16) est opposée à la partie de connexion (13), et en ce que la cavité (15) se rétrécit en direction de la partie de connexion, et présente sur sa surface intérieure, un profil en forme de sapin (17, 18, 19).

3. Sonde de guidage selon la revendication 1 ou 2, caractérisée en ce que la douille de connexion par pincement (12) est fabriquée en métal, d'un seul tenant avec la partie de connexion (13), sous forme de broche, et est disposée dans un boîtier de matière plastique (11) de forme tubulaire, de l'élément de contact (10), et en ce que le boîtier (11) présente, à une extrémité, un embout en forme de douille (20), dont la paroi périphérique entoure à distance, la partie de connexion (13).

4. Sonde de guidage selon la revendication 3, caractérisée en ce que le boîtier (11) présente, à son extrémité voisine de l'ouverture d'insertion (16) de la cavité (15), un embout tubulaire (22) comportant un canal rectiligne (28) destiné au guidage de l'extrémité (31) du fil (32), éloignée du patient.
